# EUROPÄISCHE PATENTSCHRIFT

(11) **EP 0 626 534 B1**
(45) Veröffentlichungstag und Bekanntmachung des Hinweises auf die Patenterteilung: **05.06.1996**
(21) Anmeldenummer: 94103771.5
(22) Anmeldetag: 11.03.1994
(51) Int. Cl.: F16L 3/22, A61M 5/14, H02G 3/26

(54) **Haltevorrichtung für Schlauch- und andere Leitungen an medizinischen Einrichtungen sowie im Bereich der Kranken- und Altenpflege**
Holder for tubes and other conduits connected to medical equipment in the field of nursing and care for the elderly
Dispositif de support pour tuyaux et autres conduits reliés à des équipements dans le domaine des soins pour malades et personnes agées

(30) Priorität: 23.04.1993 DE 4313329; 16.11.1993 DE 4338900
(43) Veröffentlichungstag der Anmeldung: 30.11.1994
(73) Patentinhaber: P.C. TURCK GmbH & Co. KG, 58507 Lüdenscheid (DE)
(72) Erfinder: Schneider, Eberhard, D-58553 Halver (DE)
(74) Vertreter: Patentanwälte Ostriga & Sonnet

(56) Entgegenhaltungen:
- DE-U- 9 306 143
- FR-A- 2 350 717
- GB-A- 576 759
- US-A- 3 747 166
- US-A- 4 707 906

## Beschreibung

Die Erfindung bezieht sich auf eine Vorrichtung zur Halterung von Leitungen wie Schläuchen, elektrischen Kabeln u.dgl., an Einrichtungen der Medizintechnik sowie Einrichtungen der Kranken- und Altenpflege wie Betten, Bettgestellen, Patientenkleidung od. dgl.

In Krankenhäusern, Altenpflegeheimen od.dgl. werden medizinische Leitungen wie Infusionsschläuche, Blutübertragungsschläuche und auch elektrische Leitungen, z.B. Telefonkabel oder die Anschlußleitung von Ruftastern provisorisch an einem geeigneten Ort gehalten. So werden solche Leitungen mittels Heftpflaster an Betten, medizinischen Apparaten od.dgl. angeklebt oder mit einer Sicherheitsnadel am Bettlaken angeklammert. Man verwendet solche Haltevorrichtungen wie sie gerade zur Hand sind. Ihre Anwendung kann nur als notdürftig bezeichnet werden.

Der Erfindung liegt deshalb die Aufgabe zugrunde, eine Haltevorrichtung der im Oberbegriff des Anspruchs 1 näher umrissenen Art verfügbar zu machen, die aus nur wenigen Teilen besteht, einfach handhabbar ist und eine sehr sichere und funktionsgerechte Halterung der betreffenden Leitung gewährleistet.

Die Erfindung löst diese Aufgabe durch ein die Leitung zugentlastet aufnehmendes Leitungshalteteil, ein mit diesem um eine senkrecht zur Leitungsaufnahme des Leitungshalteteils verlaufende Achse frei drehbar, jedoch axialfest verbundenes Kupplungsstück und durch ein Befestigungsteil, welches mit einem drahtartigen Abschnitt versehen ist, der sowohl entlang seiner Haupterstreckung frei hin und her beweglich als auch um seine Haupterstreckungsrichtung frei schwenkbar in einer ösenartigen Aufnahme des Kupplungsstücks aufgenommen ist.

Anders als bei den herkömmlichen Halterungsmitteln, welche jeweils sowohl die Leitung halten als auch eine Befestigungsfunktion ausüben, sind hier drei Teile vorgesehen, die sich in geschickter Zuordnung zueinander Aufgaben teilen. Das erste Teil, das Leitungshalteteil, dient unmittelbar zum Anschluß an die Leitung, und das zweite Teil, das Befestigungsteil, zur Anbringung der Haltevorrichtung an einem Fixpunkt. Diese beiden Teile sind nun über das dritte Teil, das Kupplungsstück, derart raumgelenkig miteinander verbunden, daß die Halterung insgesamt alle notwendigen Freiheitsgrade besitzt, um zu verhindern, daß die Leitung beschädigt, in ihrer wie gewünscht eingerichteten Verlegung gestört oder sonstwie in Mitleidenschaft gezogen werden kann. Dies ist insbesondere bei Infusions- und Blutleitungen von gravierender Bedeutung.

Eine wesentliche Besonderheit dieser Haltevorrichtung besteht also in der Art und Weise, wie die vollständige Raumbeweglichkeit der gehaltenen Leitung zum Fixpunkt der Befestigung realisiert ist. Eine 360^{o}-Beweglichkeit der Leitung um eine quer zu ihrer Längserstreckung verlaufende Achse wird durch die Drehverbindung zwischen Leitungshalteteil und Kupplungsstück garantiert. Dabei ist die Leitung selbst zugentlastet vom Leitungshalteteil aufgenommen. Die Leitung kann somit bezüglich der Drehachse frei hin und her pendeln, jedoch in Längsrichtung nicht verrutschen, so daß sie definiert und bestimmungsgemäß am Platze gehalten ist. Die Drehbeweglichkeit zwischen dem Leitungshalteteil und dem Kupplungsstück um eine Drehachse herum läßt sich insbesondere in Form einer Steckrastzapfenverbindung sehr leicht realisieren, zumal, wenn die Teile aus Kunststoff gespritzt sind, so daß sich die Steckrastmittel bei der Herstellung ohne weiteres mit anformen lassen.

Die weiteren Freiheitsgrade der voll beweglichen Zuordnung des Leitungshalteteils zum Befestigungsfixpunkt des Befestigungsteil am Bettgestell, am Bettzeug, an der Patientenkleidung oder wo auch immer dies gewünscht und sinnvoll ist, wird zwischen dem Befestigungsteil und dem Kupplungsstück realisiert, wobei diese Verbindung so ausgelegt ist, daß hin- und hergehende Pendel- wie auch Schwenkbewegungen bezüglich der restlichen Raumachsen gewährleistet werden.

Vorteilhaft kann es sein, wenn zwischen dem Leitungshalteteil und dem Befestigungsteil eine lösbare Steckverbindung vorgesehen ist. Dies ist beispielsweise sehr praktisch beim Bettenwechsel und auch dann, wenn das Befestigungsteil, z.B. in der Ausführung als Klebeplatte, dauerhaft an einem Gegenstand angebracht bleiben soll.

Um ein versehentliches Trennen des Kupplungsstücks vom Leitungshalteteil auszuschließen, wird eine Steckrichtung der lösbaren Steckverbindung quer zur Drehachse bevorzugt.

Sonstige vorteilhafte und zweckmäßige weitere Ausgestaltungen der Erfindung sind Gegenstand der übrigen Ansprüche und ergeben sich auch aus der nachfolgenden Beschreibung der Erfindung anhand mehrerer in den Zeichnungen dargestellter Ausführungsbeispiele. In den Zeichnungen zeigen:
- Fig. 1: wesentliche Teile eine Haltevorrichtung entsprechend einer ersten Ausführung in perspektivischer Ansicht,
- Fig. 2: eine hinsichtlich des Leitungshalteteils variierte Ausgestaltung,
- Fig. 3 und 4: zwei weitere Ausführungsformen des Leitungshalteteils,
- Fig. 5 bis 7: verschiedene Ausführungsformen von Befestigungsteilen und
- Fig. 8 und 9: eine weitere Ausführung ähnlich Fig. 1, bei der Leitungshalteteil und Kupplungsstück nach Steckrastverbindung miteinander nicht mehr ohne weiteres voneinander lösbar sind.

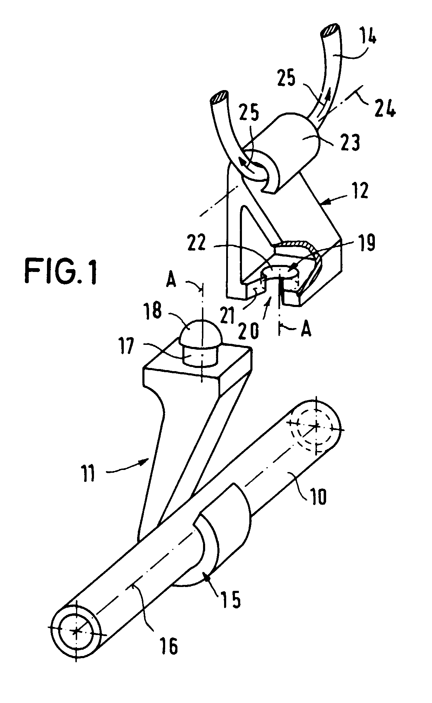

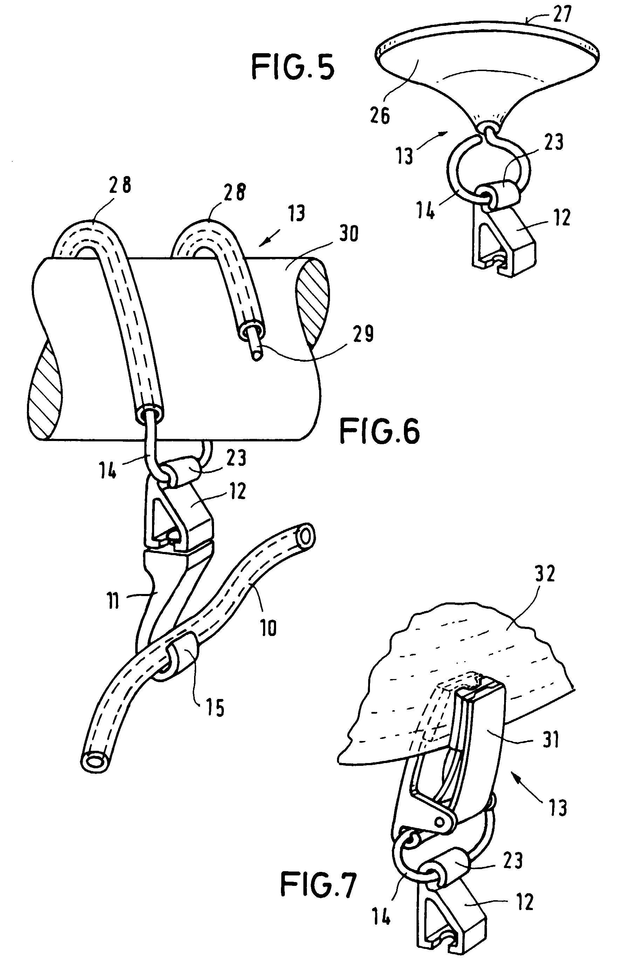

Wesentliche Bestandteile der Haltevorrichtung für eine Leitung 10, z.B. einen Infusionsschlauch, eine Bluttransportleitung oder auch ein elektrisches Kabel, sind ein Leitungshalteteil 11, ein Kupplungsstück 12 und ein Befestigungsteil 13 (Fig. 5 bis 7), von dem in Fig. 1 lediglich ein drahtbügelförmiger Abschnitt 14 als Anschlußstück dargestellt ist.

Das Leitungshalteteil 11 weist an einem Ende eine Halterung 15 zur kraft- bzw. klemmschlüssigen Aufnahme der Leitung 10 auf. Die Halterung 15 ist als an der Seite spaltoffene Öse ausgebildet, die den Mantel der Leitung 10 um mehr als 180° schützend umfaßt. Die Leitung 10 kann quer in die offene Öse eingeclipst werden. Zweckmäßigerweise sollte die Leitung 10 durch Klemm- oder leichten Kraftschluß in dieser Halterung festgehalten sein, damit sie in Längsrichtung nicht unwillentlich verschoben werden kann. Ein Klemmschluß kann bei flexiblen Leitungen 10 besonders einfach erzielt werden, indem der innere Ösen-Durchmesser etwas kleiner ausgeführt wird als der Umfang der Leitung 10.

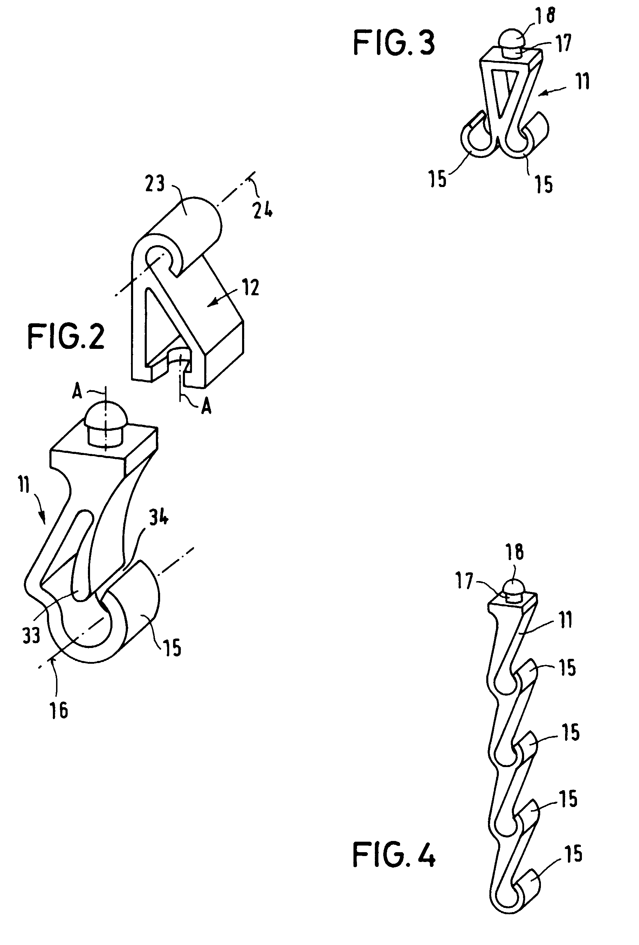

An seinem anderen Ende besitzt das Leitungshalteteil 11 einen Zapfen 17 mit einem verdickten Kopf 18. Die Längsmittelachse dieses Zapfens 17 bildet eine Drehachse A für das Kupplungsstück 12, das an seinem dem Leitungshalteteil 11 zugekehrten Ende eine Rastaufnahme 19 zum - bei diesem Ausführungsbeispiel nach den Fig. 1 und 2 wiederlösbaren - Anstecken an den Zapfen 17 aufweist. Die Rastaufnahme 19 ist hier zur Querverrastung mit dem Zapfen 17 ausgebildet, so daß trotz der gewünschten Lösbarkeit der Verbindung deren axiale Zugfestigkeit in vollem Umfang gewährleistet ist. Die Rastaufnahme 19 ist schlüssellochartig ausgebildet und unterteilt sich in einen Einrastschlitz 20 mit Aufgleit- bzw. Einlaufschrägen 21 und einen Lagerdurchbruch 22. Die Schlitzweite des Einrastschlitzes 20 ist an der engsten Stelle etwas geringer als der Durchmesser Zapfens 17, der Durchmesser des Lagerdurchbruchs 22 demgegenüber etwas größer.

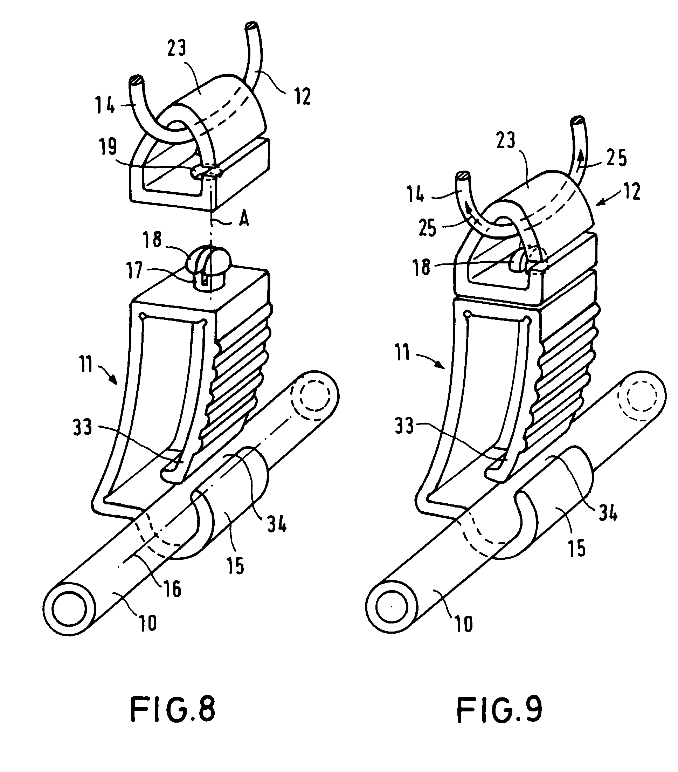

Verzichtet man auf den seitlichen Einrastschlitz und schließt die Rastaufnahme 19 zu einer geschlossen umrandeten Aufnahme für einen beispielsweise durch Schlitzung in federnde Segmente rückfedernd ausgebildeten Rastkopf 18, entsprechend dem in den Fig. 8 und 9 dargestellten Ausführungsbeispiel, so ist ebenfalls eine axiale Steckrastverbindung in Richtung der Achse A zwischen Leitungshalteteil 11 und Kupplungsstück möglich, die eine freie Drehbeweglichkeit dieser Teile zueinander ermöglicht. Um deren sichereren Zusammenhalt zu gewährleisten, ist diese Steckrastverbindung jedoch zumindest als unter üblicherweise auftretenden Zugkräften nicht trennbar ausgebildet.

Es ist daran gedacht, sowohl das Leitungshalteteil 11 als auch das Kupplungsstück 12 als Kunststoff-Spritzgießteile aus thermoplastischem, elastische Eigenschaften aufweisenden Kunststoff auszubilden. Die Aufrastung des Kupplungsstücks 12 auf das Leitungshalteteil 11 geschieht unter vorübergehender Aus- bzw. Einfederung der Kupplungsmittel. Nach vollzogener Rastverbindung ist das Kupplungsstück 12 um die Längsachse A des Zapfens 17 frei und vorzugsweise außerordentlich leichtgängig drehbeweglich mit dem Leitungshalteteil 11 verbunden.

Ähnlich wie das Leitungshalteteil 11 besitzt auch das Kupplungsstück 12 am gegenüberliegenden Ende eine der Halterung 15 ähnliche Halterung 23, die ösenartig das erwähnte Anschlußstück 14 teilumschließt, so daß dieses in die seitlich offene Öse 23 wiederlösbar eingerastet werden kann. In der Aufnahme der Öse 23 ist das Anschlußstück 14 sowohl um die Längsachse 24 der Öse 23 schwenkbar als auch in der Öse 23 in bzw. entgegen seiner Haupterstreckungsrichtung (Doppelpfeil 25) verschieblich, kann also verschiedene Pendelbewegungen ausführen.

Fig. 2 zeigt eine erweiterte Ausgestaltung des Leitungshalteteils 11 dergestalt, daß an diesem eine Federzunge 33 angeformt ist, die zum Ösenspalt 34 hinweist. Quer zur Längserstreckung 16 der Leitung 10 können Leitung 10 und Leitungshalteteil 11 daher nur dann voneinander gelöst werden, wenn die Federzunge 33 aus ihrer schlitzüberdeckenden Position ausgefedert wird.

Fig. 3 zeigt eine andere Variante eines Leitungshalteteils 11 mit zwei einander gegenüberliegenden ösenförmigen Halterungen 15, womit zwei Leitungen 10 gleichzeitig gehalten werden können. Eine Halterung für vier Leitungen in paralleler Zuordnung untereinander zeigt Fig. 4. Kombinationen und Varianten sind, wie aus diesen Darstellungen ohne weiteres ableitbar ist, in mehrfacher Hinsicht möglich.

Für unterschiedliche Anwendungszwecke können verschiedene Befestigungsteile 13 nach Art von Adaptern wahlweise verwendet werden.

In Fig. 5 ist ein Sauger dargestellt mit einem in die Öse 23 einzuclipsenden Ring als Anschlußstück 14. Man kann sich den dargestellten Sauger 26 aber auch als Klebeplatte vorstellen, deren Rückseite 27 z.B. mit einer doppelseitigen Klebefolie ausgerüstet ist.

Fig. 6 zeigt als Befestigungsteil 13 einen mit Gummi- oder Silikonschlauchabschnitten 28 als Schutz umhüllten Draht 29, der elastische, jedoch zugleich auch plastisch verformbare Eigenschaften aufweist und so den Erfordernissen entsprechend gebogen werden kann. Das Anschlußstück 14 wird bei dieser Ausführung von einem Abschnitt des Drahtes 29 selbst bereitgestellt. Mit Hilfe eines solchen Befestigungsteils 13 läßt sich eine Leitung 10 in besonders geeigneter Weise an einem Bettpfosten 30, einer Apparatestrebe od.dgl. anbringen.

In Fig. 7 ist schließlich noch eine Ausführung für ein Befestigungsteil 13 dargestellt, welches aus einem Clip 31 nach Art eines Hosenträgerclips besteht. Als drahtbügelartiges Anschlußstück 14 dient hier dasjenige Element des Clips, welches üblicherweise die Schlaufe eines Hosenträgergurtes durchgreift. Mit Hilfe dieses Befestigungsteils 13 läßt sich eine Leitung 10 schonend an Stoffen, wie z.B. einem Bettlaken 32, an der Patientenkleidung od.dgl. anbringen.

## Patentansprüche

1. Vorrichtung zur Halterung von Leitungen wie Schläuchen, elektrischen Kabeln u.dgl. an Einrichtungen der Medizintechnik sowie Einrichtungen der Kranken- und Altenpflege wie Betten, Bettgestellen, Patientenkleidung od. dgl., gekennzeichnet durch ein die Leitung (10) zugentlastet aufnehmendes Leitungshalteteil (11), ein mit diesem um eine senkrecht zur Leitungsaufnahme (15) des Leitungshalteteils (11) verlaufende Achse (A) frei drehbar, jedoch axialfest verbundenes Kupplungsstück (12) und durch ein Befestigungsteil (13), welches mit einem drahtartigen Abschnitt (14) versehen ist, der sowohl entlang seiner Haupterstreckung (Doppelpfeil 25) frei hin- und herbeweglich als auch um seine Haupterstreckungsrichtung frei schwenkbar in einer ösenartigen Aufnahme (23) des Kupplungsstücks aufgenommen ist.

2. Vorrichtung nach Anspruch 1, dadurch gekennzeichnet, daß die Verbindung zwischen dem Leitungshalteteil (11) und dem Kupplungsstück (12) als axialsteckbare, insbesondere ohne Werkzeug unlösbare, Rastzapfenverbindung (17, 18; 19) ausgebildet ist.

3. Vorrichtung nach Anspruch 1, gekennzeichnet durch Steckrichtung der Rastverbindung (17/19) quer zur Drehachse (A).

4. Vorrichtung nach Anspruch 1, dadurch gekennzeichnet, daß die Halterung (15) des Leitungshalteteils (11) und/oder die Aufnahme (23) des Kupplungsstücks (12) als den Mantel der Leitung (10) bzw. den drahtartigen Abschnitt (14) des Befestigungsteils (13) als seitenoffene Öse ausgebildet ist, in die die Leitung (10) bzw. der drahtartige Abschnitt (14) quer zu ihrer/seiner Haupterstreckung (16 bzw. 25) einclipsbar ist.

5. Vorrichtung nach Anspruch 4, dadurch gekennzeichnet, daß die Leitung (10) vom Ösenhauptabschnitt der Halterung (15) um mehr als 180⁰ umschlossen ist.

6. Vorrichtung nach Anspruch 4 oder 5, dadurch gekennzeichnet, daß der ösenartigen Aufnahme (15) ein die Leitung (10) in ihr sicherndes, lösbares Sperrorgan in Form einer am Leitungshalteteil (10) angeformten, den Ösenspalt (34) versperrende Federzunge (33) zugeordnet ist.

7. Vorrichtung nach einem der vorhergehenden Ansprüche, dadurch gekennzeichnet, daß das Leitungshalteteil (11) mehrere Halterungen (15) in Über- und/oder Nebeneinanderanordnung aufweist.

8. Vorrichtung nach Anspruch 1, dadurch gekennzeichnet, daß das Leitungshalteteil (11) und/oder das Kupplungsstück (12) ein Kunststoffspritzgießteil ist.

9. Vorrichtung nach Anspruch 1, dadurch gekennzeichnet, daß das Befestigungsteil (13) eine Klemme oder Klammer bzw. ein Clip (31) insbesondere nach Art eines Hosenträgerclips ist.

10. Vorrichtung nach Anspruch 1, dadurch gekennzeichnet, daß das Befestigungsteil (13) ein Magnet ist.

11. Vorrichtung nach Anspruch 1, dadurch gekennzeichnet, daß das Befestigungsteil (13) ein Sauger (26) ist.

12. Vorrichtung nach Anspruch 1, dadurch gekennzeichnet, daß das Befestigungsteil (13) eine Klebeplatte ist.

13. Vorrichtung nach Anspruch 1, dadurch gekennzeichnet, daß das Befestigungsteil (13) eine Schleife, Wendel, Klammer od.dgl. aus Draht (29) ist.

## Claims

1. Device for fixing lines such as tubes, electrical cables and the like to medical equipment and to facilities used in the nursing of the sick and the elderly, such as beds, bedsteads, patients' clothing, or the like, characterized by a line retaining part (11) which holds the line (10), relieved of strain, a coupling piece (12) which is joined to the line retaining part so as to be capable of rotating freely around an axis (A) running perpendicular to the line holder (15) of the line retaining part (11), but in an axially fixed manner, and by a fastening part (13) which is provided with a wire-type section (14) which is accommodated in a lug-type holder (23) of the coupling piece so as to be capable both of moving freely to and fro along a main length (double arrow 25) and of swivelling freely around the direction of its main length.

2. Device according to Claim 1, characterized in that the joint between the line retaining part (11) and the coupling piece (12) is formed as an axially inserted locking peg joint (17, 18; 19), particularly one which is not separable without a tool.

3. Device according to Claim 1, characterized by a direction of insertion of the locking joint (17; 19) which is transversal relative to the rotational axis (A).

4. Device according to Claim 1, characterized in that the holder (15) of the line retaining part (11) and/or the holder (23) of the coupling piece (12) are formed as laterally open lugs [enclosing], respectively, the circumferential surface of the line (10) and the wire-type section (14) of the fastening part (13), into which the line (10) and the wire type section (14) respectively are clipped transversely relative to the main length (16 or 25) of the line/section.

5. Device according to Claim 4, characterized in that the lug main section of the holder (15) encloses the line (10) by more than 180°.

6. Device according to Claim 4 or 5, characterized in that the lug-type holder (15) includes a releasable locking element within which the line (10) is secured, in the form of spring tongue (33), formed on the line retaining part (11), which obstructs the lug slot (34).

7. Device according to any one of the preceding Claims, characterized in that the line retaining part (11) has several holders (15) arranged above and/or adjacently to one another.

8. Device according to Claim 1, characterized in that the line retaining part (11) and/or the coupling piece (12) is a plastics injection moulded part.

9. Device according to Claim 1, characterized in that the fastening part (13) is a clamp, cramp or clip (31), particularly one in the style of a trouser braces' clip.

10. Device according to Claim 1, characterized in that the fastening part (13) is a magnet.

11. Device according to Claim 1, characterized in that the fastening part (13) is a suction pad (26).

12. Device according to Claim 1, characterized in that the fastening part (13) is an adhesive disc.

13. Device according to Claim 1, characterized in that the fastening part (13) is loop, coil, hook, or the like, made from wire (29) .

## Revendications

1. Dispositif pour retenir des conduites, comme des tuyaux, des câbles électriques et similaires sur des dispositifs de la technique médicale, ainsi que sur des dispositifs destinés aux soins des malades et des personnes âgées, comme des lits, des armatures métalliques de lits, des vêtements de patients ou similaires, caractérisé par un élément de retenue de la conduite (11) qui reçoit la conduite (10) sans qu'elle ne soit soumise à une traction, par une pièce d'accouplement (12) reliée à celui-ci en pouvant tourner librement autour d'un axe (A) s'étendant perpendiculairement au logement (15) de l'élément de retenue de la conduite (11) qui est destiné à la conduite, mais d'une manière rigide dans le sens axial, et par un élément de fixation (13) pourvu d'une partie (14) analogue à un fil, celle-ci étant reçue dans un logement analogue à un crochet (23) de la pièce d'accouplement en pouvant aussi bien se déplacer librement en va-et-vient dans le sens de sa direction principale (flèche double 25) que pivoter librement autour de sa direction principale.

2. Dispositif selon la revendication 1, caractérisé par le fait que la liaison entre l'élément de retenue de la conduite (11) et la pièce d'accouplement (12) est réalisée sous la forme d'une liaison par un tenon à encliquetage (17, 18 ; 19) qui peut être enfoncé axialement et qui, en particulier, ne peut pas être détaché sans outils.

3. Dispositif selon la revendication 1, caractérisé par une direction d'enfoncement de la liaison par encliquetage (17/19) qui est transversale par rapport à l'axe de rotation (A).

4. Dispositif selon la revendication 1, caractérisé par le fait que l'organe de retenue (15) de l'élément de retenue de la conduite (11) et/ou le logement (23) de la pièce d'accouplement (12) est réalisé sous la forme d'un crochet qui entoure la surface latérale de la conduite (10) ou la partie (14) analogue à un fil de l'élément de fixation (13), respectivement, qui est ouvert latéralement et dans lequel la conduite (10) ou la partie (14) analogue à un fil, respectivement, peut être encliquetée transversalement par rapport à sa direction principale (16, respectivement 25).

5. Dispositif selon la revendication 4, caractérisé par le fait que la conduite (10) est entourée sur plus de 180° par la partie principale du crochet de l'organe de retenue (15).

6. Dispositif selon la revendication 4 ou 6, caractérisé par le fait qu'au logement analogue à un crochet (15) est associé un organe de blocage amovible qui arrête la conduite (10) dans ce logement et qui est réalisé sous la forme d'une languette élastique (33), celle-ci étant formée sur l'élément de retenue de la conduite (11) et fermant la fente (34) du crochet.

7. Dispositif selon l'une des revendications précédentes, caractérisé par le fait que l'élément de retenue de la conduite (11) présente plusieurs organes de retenue (15) superposés et/ou juxtaposés.

8. Dispositif selon la revendication 1, caractérisé par le fait que l'élément de retenue de la conduite (11) et/ ou la pièce d'accouplement (12) est une pièce en matière plastique moulée par injection.

9. Dispositif selon la revendication 1, caractérisé par le fait que l'élément de fixation (13) est une pince ou un organe de serrage ou une agrafe (31), respectivement, en particulier à la manière d'une pince de bretelles.

10. Dispositif selon la revendication 1, caractérisé par le fait que l'élément de fixation (13) est un aimant.

11. Dispositif selon la revendication 1, caractérisé par le fait que l'élément de fixation (13) est une ventouse (26).

12. Dispositif selon la revendication 1, caractérisé par le fait que l'élément de fixation (13) est une plaque adhésive.

13. Dispositif selon la revendication 1, caractérisé par le fait que l'élément de fixation (13) est une boucle, un organe hélicoïdal, une pince ou un organe similaire qui est constitué par un fil (29).
